Europäisches Patentamt

European Patent Office

Office européen des brevets

(11) Publication number: **0 429 813 A1**

## EUROPEAN PATENT APPLICATION

(21) Application number: **90119486.0**

(22) Date of filing: **11.10.90**

(51) Int. Cl.5: **B01J 3/03**, F16J 13/00, A61L 2/04

(30) Priority: **27.10.89 IT 2215589**

(43) Date of publication of application:
**05.06.91 Bulletin 91/23**

(84) Designated Contracting States:
**AT BE CH DE DK ES FR GB GR LI LU NL SE Bulletin**

(71) Applicant: **FEDEGARI AUTOCLAVI SPA**
**S.S. 235 KM 8**
**I-27010 Albuzzano (PV)(IT)**

(72) Inventor: **Fedegari, Fortunato**
**Vicolo P. Terenzio, 2**
**I-27100 Pavia(IT)**

(74) Representative: **Michelotti, Giuliano et al**
**c/o SAIC BREVETTI S.r.l. Viale Bianca Maria**
**15**
**I-20122 Milano(IT)**

(54) **Autoclave door with minimum opening encumbrance.**

(57) Autoclave door (12) combining a lateral movement, for engaging and disengaging its own frame, with a rotating movement around a pintle axis (20), parallel to a side thereof, to allow substantially book closing and opening operations requiring minimum encumbrance and providing simplicity and seal safety.

Tav.I

Fig.1

## AUTOCLAVE DOOR WITH MINIMUM OPENING ENCUMBRANCE

Present invention refers to autoclave doors and, specifically, to autoclave doors provided with means assuring a lateral movement for engagement and disengagement with respect to a frame and rotary movement around a pintle axis.

The autoclaves, paricularily those for sterilizers, are well known since long time as consisting of a room, provided with airproof walls, expected to resist very high pressures, and possibly provided with proper thermal insulating means, wherein high temperature and high pressure fluids, such as steam, are admitted for providing sterilization of chemicals, food matters, medical materials, etc.

In food, pharmaceutic, medical industries and the like, are needed larger and larger autoclaves for providing to bulky sterilizations of higher and higher quantities of materials.

One of the hardest problems met in manufacturing large autoclaves consists of the admission doors, which must have the largest the possible size, because said doors must resist possibly very high pressures, allow an absolutely tight seal and provide perfect hygenic conditions.

There are doors having a so called book opening, i.e provided with a plurality of pintles along a side therof, allowing the rotation thereof around an axis parallel to said side. This kind of opening, which is subsantially similar to the common house doors, allows to contain the door encumbrance within a region delimitated by a circle arc in front of the autoclave opening. However, when the door must stand very high pressures, must be provided with closing means of the kind having coming out bars, as in strong-room doors, compelling to incorporate in the wing bar mechanisms requiring an abundant lubrication, for example by means of grease making difficult or precarious to meet the hygienic conditions required by most autoclaves.

To obviate this problem, it has been devised to use laterally sliding doors, either horizontally or vertically, provided with sealing means, such as a sealing gasket, coming out from a frame for providing the seal between said door and the corresponding frame, as depicted in the Italian Utility Model Patent No. 189,131 having title "Autoclave sealing device" filed on November 10, 1982 at the name of the present Applicant, whose specication is here incorporated by reference.

This system works rather well, because to fasten a sliding door to the frame just some brackets mounted on the door and engaging corresponding brackets mounted on the frame are sufficient, and to provide a seal between door and frame the use of the sealing device depicted in the above mentioned Utility Model Patent is sufficient.

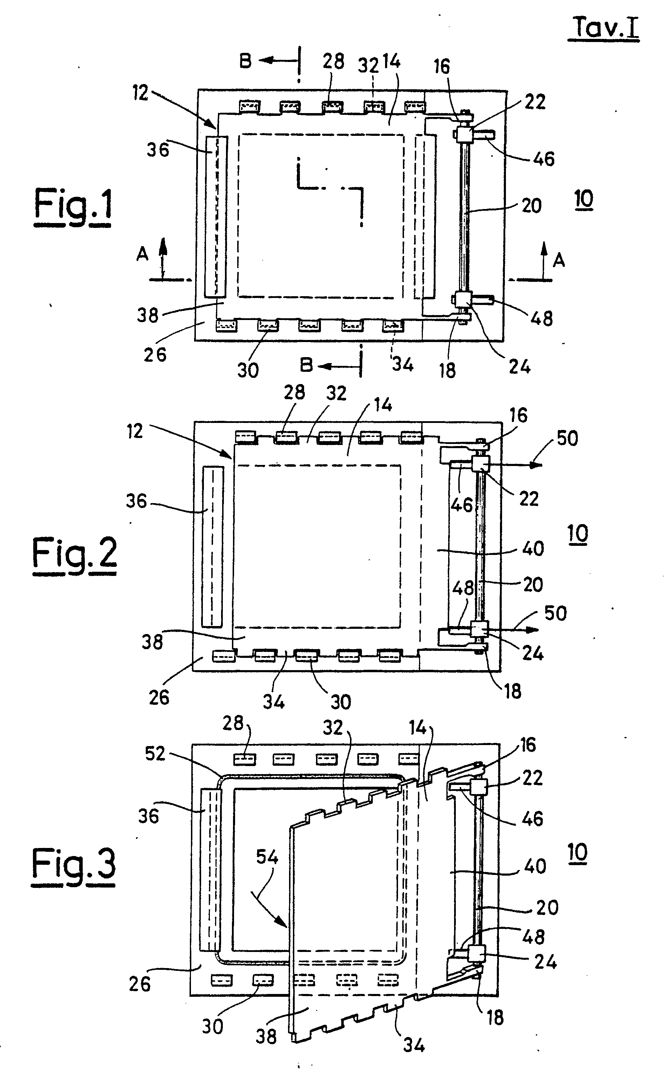

However, it remain the serious drawback inherent to any sliding door, i. e. a large lateral encumbrance due to the fact that the door, completely sliding along a side, covers an area substantially equal to the admittance area to the autoclave, as by figure 3 of the above mentioned Patent, giving encumbrance problems, to be taken into account in setting up a plurality of autoclaves in a room, and imposing serious limits in the use tereof.

To obviate the above drawback must be used a door provided with limited lateral sliding movement for engaging and disengaging its bracket means with corresponding bracket means on a door frame and rotary movement around an axis parallel to a side thereof for completely closing and opening said door, further providing, for assuring the seal, means similar to those depicted in the above mentioned Utility Model Patent.

Specifically, said lateral movement is provided along a straight line in a direction perpendicular to the rotary movement axis parallel to one of door sides.

Alternatively, said lateral movement is provided along a straight line parallel to said rotary movement axis.

Further alternatively, said lateral movement is made along a straight line in an inclined direction substantially parallel to a diagonal of the door itself.

Further, the above mentioned lateral movement can be made, instead than along a straight line, along a circle arc having such a long radius to substantially approssimate a straight segment, as it is often practic to make rotary movements also to provide said lateral movements.

Particularly, said door, of generally rectangular shape, is provided with comb arranged protrusions engaging corresponding brackets, similarly comb arranged, along sides parallel to the lateral movement thereof, so that in a closing operation said protrusions engage said brackets and in an opening operation the protrusions clear said brackets, and along the sides perpendicular to said lateral movement is provided, on the side upstream the movement, with a continuous bracket fastened to the frame and enageable in the corresponding door side and, on the side downstream the movement, a bracket incorporated in the door engageable in a corresponding bracket fastened to said frame.

Alternatively, for substantially diagonal lateral movements, are provided along the sides upstream the movement continuous brackets fastened to the frame and, along the sides downstream the movement, continuous brackets incorporated in the door and engageable with corresponding brackets fastened to said frame.

In a particularly preferred embodiment of the present invention, the lateral movement is provided by mechanical motor means, while the rotary closing and opening movement of the door is carried out manually.

In an alternate embodiment, both the lateral and rotary movements are provided by mechanical motor means.

Preferably, said mechanical motor means, providing both the lateral and the rotary movement, comprise two different motors for the two different movements.

Alternatively, both the lateral and the rotary movement are provided by just one motor providing first the lateral movement, then the rotary movement, in case of door opening, and viceversa, in case of closure thereof.

The features and the advantages of the present invention will be more apparent from the herebelow detailed description of embodiments thereof, given in an exemplifying and not limiting way, provided with the enclosed drawings, wherein:

Figure 1 is a schematical front view of a first embodiment of the present invention, providing lateral movement perpendicular with respect to the rotary axis of the door pintles, in the condition of closed and locked door;

Figure 2 is a schematical front view of said first embodiment of the present invention in the condition of closed but unlocked door;

Figure 3 is a schematical, partial perspective of the door itself according to the first embodiment during an opening movement;

Figure 4 is a schematical front view of a second embodiment of the present invention, providing lateral movement parallel with respect the rotary axis of the door pintles, in the condition of closed and locked door;

Figure 5 is a schematical front view of said second embodiment of the present invention in the condition of closed but unlocked door;

Figure 6 is a schematical, partial perspective, of the door itself in the second embodiment during an opening movement;

Figure 7 is a cross-section schematic view taken along the A-A line of figure 1;

Figure 8 is a cross-section schematic view taken along the line B-B of figure 1;

Figure 9 is a cross-section schematic view taken along the line C-C of figure 4;

Figure 10 is a cross section schematic view taken along the line D-D of figure 4;

Figure 11 is a schematic front view of a third embodiment of the present invention, providing lateral movement in a direction substantially diagonal with respect to the door itself in the closed and locked condition;

Figure 12 is a schematic front view of said third embodiment of the invention in the condition of closed but unlocked door;

Figure 13 is a schematic, partially perspective of the door itself, according to the third embodiment of the invention, during an opening movement;

Figure 14 is a schematic cross-section view taken along the line A-A of figure 11;

Figure 15 is a cross-section schematic view taken along the line B-B of figure 11.

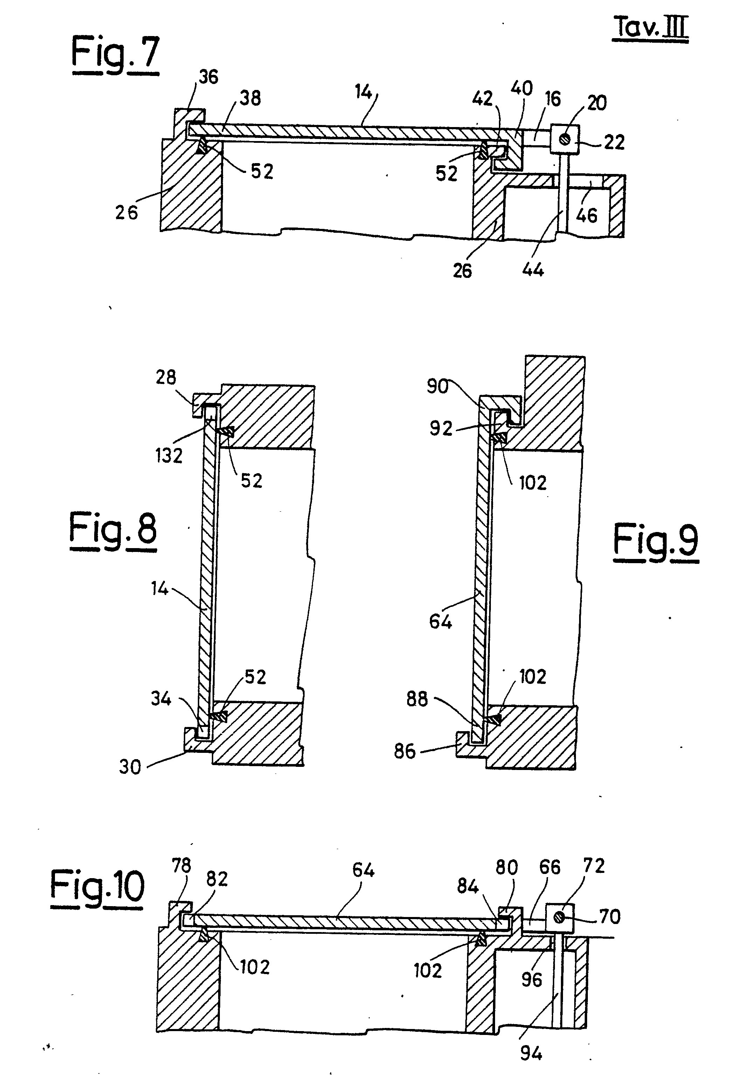

Referring to figures 1-3 and 7, 8, depicting a first embodiment of the invention, it is to see that an autoclave 10 is frontally closed by a door 12 comprising a wing 14 and pintles 16 and 18 rotatable around an axis 20, sustained by bearing supports 22 and 24. The above mentioned wing 14 is provided with fastening means to a door frame 26 consisting of bracket sets 28 and 30 perpendicularly protruding along the upper and lower sides of said frame 26 and engageable in corresponding protrusions 32 and 34, coming out of respective upper and lower sides of said wing 14, of a right bracket 36 protruding, for example, from the left side of the door frame 26 and engaging the corresponding left side 38 of the wing 14 itself and of a right bracket 40, integrally formed with the side, for example the right side, of the wing 14 and engaging a counterbracket 42 integrally formed with the door frame 26 (see particularly figure 7).

To allow the first lateral sliding movement of the door 12, in order to clear the wing 14 from the fastening means, the bearing supports 22 and 24 are provided with sliding means, such as a rod 44, visible in figure 7 as connected with the support 22, movable within slots 46 and 48 according to a direction indicated by arrows 50.

As particularly visible in figure 3, a gasket 52, of the kind disclosed and claimed in the above mentioned Italian Utility Model Patent No. 189,131, is used to provide seal between the frame 26 and the wing 14 according to the provisions indicated in the specifications of the above patent.

The operation of the first embodiment of the present invention is the following:
when the wing 14 of the door 12 has to be closed and fastened, it is in the condition depicted in figures 1, 7 and 8 with the right brackets 28 engaging the protrusions 32 and the brackets 30 engaging the protrusions 34 on the top and bottom sides, respectively, of the wing 14, the right bracket 36 engaging the side 38 of the wing itself and the bracket 40, integrally formed with the wing 14 itself, engaging the counterbracket 42 integrally formed with the frame 26, the gasket 52 resulting protruded to get in touch with the internal wall of the wing 14, in order to permit the seal for the internal room of the autoclave 10.

When said wing 14 is to be opened, it is first withdrawn the gasket 52 in the way depicted in the above mentioned Italian Utility Model Patent No. 189,131, then the whole wing 14 is laterally slipped in the direction indicated by the arrows 50, allowing the right brackets 28 to clear the protrusions 32, the brackets 30 to clear the protrusions 34, the bracket 36 to clear the side 38 and the bracket 40 to clear the counterbracket 42, as particularly depicted in the figure 2.

Presently, being all the fastening means of the wing 14 cleared, the door can be completely open by making rotate the wing 14 itself in the direction indicated by the arrow 54 in figure 3.

The closing operation of the door 12 is carried out by obviously inverting the above disclosed operation sequence, i.e. starting from the open door depicted in figure 3 to come to the closed and engaged door depicted in the figures 1, 7 and 8 with the right brackets 28, 30, 36 and 42 engaged and the gasket 52 protruded in order to provide closure and hermetic seal to the door itself.

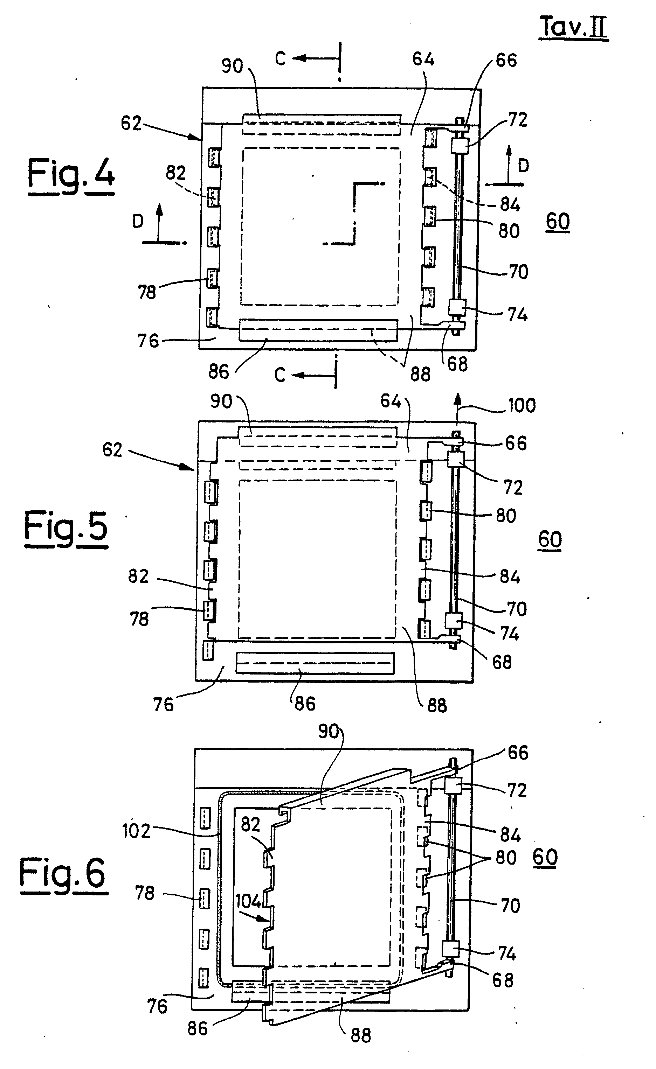

Referring now to figures 4-6 and 9, 10 depicting a second embodiment of the invention, it is to see that an autoclave 60 is front-closed by a door 62 comprised by a wing 64 and pintles 66 and 68 rotatable around an axle 70 supported by bearing supports 72 and 74. Said wing 64 is provided with fastening means to a door frame 76 consisting of bracket sets 78 and 80 perpendicularly protruding along the right and left sides of said frame 76 and engageable in correspondig protrusions 82 and 84, coming out of the right and left sides, respectively, of said wing 64, of a right bracket 86 protruding, for example from the bottom side of door frame 76 and engaging the corresponding bottom side 88 of the wing 64 itself and a right bracket 90 integrally formed with the side, for exampe the top side, of the wing 64 and engaging a counterbracket 62 integrally formed with the door frame 76 (see specifically figure 9).

To allow the first sliding movement to the door 62, in order to have the wing 64 cleared by the fastening means, the bearing supports 72 and 74 are provided with moving means, such as a rod 94 visible in figure 9 connected with the support 72, movable within slots , such as the slot 96, in accordance with a direction indicated by an arrow 100.

As particularly visible in figure 6, a gasket 102, of the kind disclosed and claimed in the above mentioned Italian Patent for Utility Model No. 189,131, is used to provide the seal between the frame 76 and the wing 64 according to the provisions specified in the disclosure of the mentioned patent.

The operation of the second embodiment of the present invention is the following:
when the wing 64 of the door 62 must be closed and fastened, it is in the condition depicted in figures 4, 9 and 10 with the right brackets 78 engaging the protrusions 82 and the brackets 80 engaging the protrusions 84 on the sides left and right, respectively, of the wing 64, the right bracket 86 engaging the side 88 of the wing itself and the bracket 90, integrally formed with the wing 64 itself, engaging the counterbracket 92 integrally formed with the frame 76, the gasket 102 resulting protruded to touch the internal wall of the wing 64 in order to allow the seal for the internal room of the autoclave 60.

When said wing 64 is wanted to be opened,it is first withdrawn the gasket 102 in the way depicted in the above mentioned Italian Patent for Utility Model No. 189,131 and then the whole wing 64 is laterally slipped in the direction indicated by the arrow 150, allowing the right brackets 78 to clear the protrusions 82, the brackets 80 to clear the protrusions 84, the bracket 86 to clear the side 88 and the bracket 90 to disengage the counterbracket 92, as particularly depicted in figure 5.

Presently, being all the constraints of the wing 64 cleared, the door can be completely open rotating the wing itself in the direction indicated by the arrow 104 in figure 6.

The closure operation of the door 62 is obviously carried out by inverting the above depicted operation sequence, i.e. by starting from the open door shown in figure 6 to arrive to the closed and engaged door depicted in the figures 4, 9 and 10 with the right brackets 78, 80, 86 and 92 engaged and the gasket 102 protruded in order to provide closure and hermetic seal to the door itself.

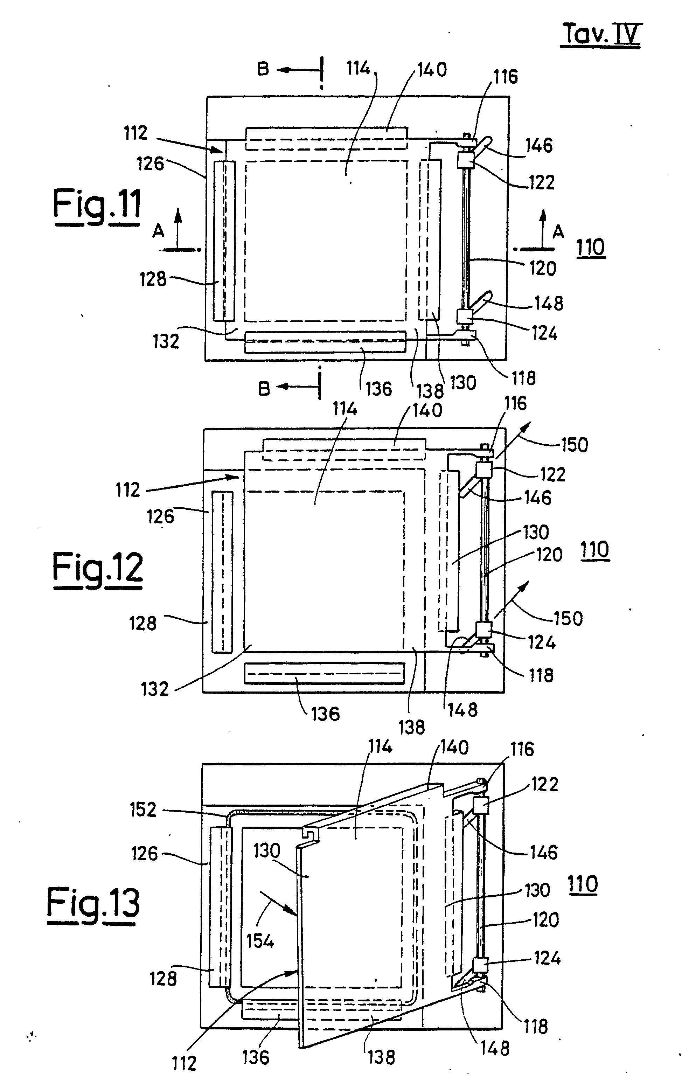

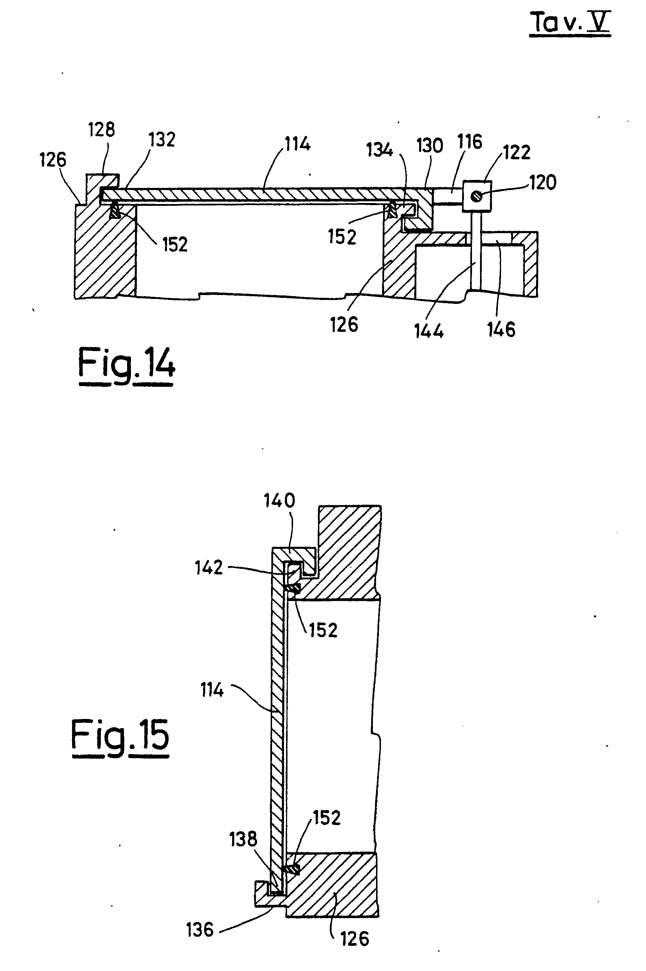

Referring now to figures 11-15, depicitng a third embodiment of the invention, it is to see that an autoclave 110 is front-closed by a door 112 comprised by a wing 114 and pintles 116 and 118 rotatable around an axle 120 supported by bearing supports 122 and 124. Said wing 114 is provided with fastening means to a door frame 126, such as a left right bracket 128, fastened to the door frame 126, and a right bracket 130, integrally built with the wing 114, the right bracket 128 engaging a corresponding wing side 132 and the right bracket 130 engaging a counterbracket 134 integrally built with the door frame 126 (see figure 14) as well as a bottom bracket 136, fastened to the door frame 126, and a top right bracket 140, integrally built with the wing 114, the right bracket 136 engaging a corresponding frame side 138 and the right bracket 140 engaging a counterbracket 142 integrally built with the door frame 126 (see figure 15).

To allow the first sliding movement of the door 112, in order to clear the wing 114 from the fastening means, the bearing supports 122 and 124 are provided with moving means, such as a rod 144 visible in figure 14 connected with the support 122, movable into slots 146 and 148 according to the direction indicated by substantially diagonal arrows 150. As particularly visible in figure 13, a gasket 152, of the kind disclosed and claimed in the above mentioned Italian Patent for Utility Model No. 189,131, is used to provide the seal between the frame 126 and the wing 114 according to the there indicated way.

The operation of the third embodiment of the present invention is the following:
when the wing 114 of the door 112 must be closed and fastened, it is in the condition depicted in figures 11, 14 and 15 with the right bracket 128 engaging the left side 132 of the wing and the right bracket 130, integrallly formed with the wing 114 itself, engaging the counterbracket 134 integrally formed with the frame 126, the right bracket 136 engaging the bottom side 138 of the wing 114 and the top bracket 140, integrally formed with the wing, engaging the counterbracket 142 integrally formed with the frame 126, the gasket 152 resulting protruded to get in touch with the internal wall of the wing 114, in order to allow a seal for the internal room of the autoclave 110.

When it is desired to open said wing 114, first the gasket 152 is withdrawn in the way depicted in the above mentioned Italian Patent for Utility Model No. 189,131 and then the whole wing 114 is diagonally slipped in the direction indicated by the arrows 150 allowing the right bracket 128 to clear the wing side 132, the right bracket 136 to clear the wing side 138, the bracket 130 to disengage the counterbracket 134 and the bracket 140 to disengage the counterbracket 142, as particularly depicted in figure 12. Presently, being all the constraints of the wing 114 cleared, the door can be completely open by making rotate the wing 114 itself in the direction indicated by the arrow 154 in figure 13. The closure operation of the door 112 is obviously carried out by inverting the above depicted operation sequence, i.e. by starting from the open door shown in figure 13 to arrive at the closed and engaged door depicted in the figures 11, 14 and 15 with the brackets 128, 130, 136 and 142 engaged and the gasket 152 protruded, in order to provide closure and hermetic seal to the door itself.

What has been here above disclosed depicts some embodiment examples of the invention, not to be anyway construed in a limiting sense, and it will be easily possible to those skilled in this art to find obviously devisable changements and equivalent and adjoining variations, to be all here covered.

For example, the sliding movements can be carried out along circle arcs in stead of right segments simply by providing the same movements by means of devices rotating along limited angles, said devices consisting either of rotating motors operating through gear sets, or linear motors operating through racks and gears, or rods and cranks operating with lever arms providing the sliding movements for the wing of said door.

The rotating movements to provide the complete opening of the door can be carried out by hand or by a motor, being the motor a motor properly dedicated thereto, or the same motor providing the sliding movement.

## Claims

1. Limited opening encumbrance autoclave door, comprising a wing (14,64, 114) movable with respect to a frame (26, 76, 126) fastened to walls of said autoclave, characterized by providing a limited lateral sliding movement for engaging and disengaging bracket means on said wing (14,64, 114) with corresponding bracket means on said door frame (26, 76 126) and a rotary movement around an axis (20,70, 120) parallel to one of the sides of the door itself for a complete closure or opening thereof, further using known gasket means to provide for a seal thereof.

2. Autoclave door, as in claim 1, characterized in that said lateral sliding movement is carried out along a right line (50) perpendicular with respect to that of the axis (20) of said rotary movement parallel to one of the sides of the door.

3. Autoclave door, as in claim 1, characterized in that said lateral movement is carried out along a right line (100) parallel in direction to that of the axis (70) of rotary movement parallel to one of the sides of the door.

4. Autoclave door, as in claim 1, characterized in that said lateral movement is carried out along a right line (150) in an inclined direction substantially parallel to a diagonal of the door itself.

5. Autoclave door, as in claims 2 to 4, charcterized in that the lateral sliding movements can be carried out, instead of along a right line, along a circle arc having so long a radius to substantially approach a right segment.

6. Autoclave door, as in claims 2 and 3, characterized in that said door, provided with wings (14, 64), having generally rectangular shape, is provided with comb arranged protrusions (32, 34; 82, 84) engageabe in corresponding brackets (28, 30; 78, 80), also comb arranged, along sides thereof parallel to the lateral sliding movement, so that in a closure operation said protrusions (32, 34; 82, 84) are engaged under said brackets (28, 30; 78, 80) and in an opening operation the protrusions are cleared by the brackets themselves, and along the sides of the wing (14,64) perpendicular with respect to said lateral sliding movement provides on the upstream side of the movement a continuous bracket (36,86) fastened to the door frame (26, 76) engageable in the corresponding side (38,88) of the wing (14,64) of said door and on the downstream side of the movement a bracket (40, 90) incorporated in the wing (14, 64) of said door engageable with a corresponding bracket (42,92) fastened to said door frame (26, 76).

7. Autoclave door, as in claim 4, characterized in that, for allowing lateral sliding movements of said wing (114), are used along the upstream sides of the movement continuous brackets (128, 136) fastened to the door frame (126) and along the downstream sides of the movement continuous brackets (130, 140) incorporated in the door wing (114) engageable with corresponding brackets (134, 142) fastened to said door frame (126).

8. Autoclave door, as in claims 6 and 7, characterized in that the lateral sliding movement is provided by mechanical motorized means, while the rotary opening and closure movement is manually carried out.

9. Autoclave door, as in claims 6 and 7, characterized in that both the lateral sliding movement and the rotary movement are provided by mechanical motorized means.

10. Autoclave door, as in claim 9, characterized in that said mechanical motorized means, providing both the lateral sliding movement and the rotary movement, comprise two different motors for the two different movements.

11. Autoclave door, as in claim 9, characterized in that both the lateral sliding movement and the rotary movement are provided by just one motor operating on a mechanism providing first the lateral sliding movement and then the rotary movement, in case of door opening and viceversa, in case of closure thereof.

Fig.1

Fig.2

Fig.3

Fig.4

Fig.5

Fig.6

Tav. III

Fig.7

Fig.8

Fig.9

Fig.10

## Fig.11

110

## Fig.12

110

## Fig.13

110

Tav. V

Fig.14

Fig.15

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | US-A-4 932 160 (SPERKO)(12-06-1990)<br>* Abstract; column 1, line 53 - column 2, ine 12; column 2, lines 29-57; column 3, line 4 - column 4, line 27; figures 2-4C *<br>- - - | 1,2 | B 01<br>J 3/03<br>A 61 L 2/26<br>E 05 F 7/02<br>F 16 J 13/16 |
| A | | 7-9,11 | |
| X | US-A-3 490 641 (BEECHER)<br>* Abstract; column 3, lines 5-50; figures 1A-1C; claim 1 *<br>- - - | 1,4,7 | |
| A | | 8 | |
| X | US-A-4 262 447 (SCHNEIER et al.)<br>* Abstract; column 1, lines 6-12; column 2, line 16 - column 3, line 50; claim 1; figures 1-4 *<br>- - - | 1,2 | |
| A | US-A-2 917 987 (HANSEN et al.)<br>* Column 3, lines 44-71; figures 1,2 *<br>- - - | 3 | |
| A | US-A-3 888 045 (PIEGZA)<br>* Column 1, lines 6-10; column 1, lines 32-56; figures 1,2 *<br>- - - - - | 6 | TECHNICAL FIELDS SEARCHED (Int. Cl.5)<br><br>B 01 J<br>A 61 L<br>E 05 F<br>F 16 J |

The present search report has been drawn up for all claims

| Place of search | Date of completion of search | Examiner |
|---|---|---|
| The Hague | 12 March 91 | STEVNSBORG N. |